# EUROPEAN PATENT APPLICATION

(11) **EP 4 124 353 A1**
(43) Date of publication of application: **01.02.2023**
(21) Application number: 22186527.2
(22) Date of filing: 22.07.2022
(51) Int. Cl.: A61L 9/20

(54) **DEVICE FOR SANITIZING AIR BY ULTRAVIOLET RADIATION**

(30) Priority: 28.07.2021 IT 202100020087
(71) Applicant: NVK DESIGN di Natasha Calandrino, 20159 Milano (IT); Istituto Nazionale di Astrofisica, 00136 Roma (IT)
(72) Inventor: Calandrino Van Kleef, Natasha, 20159 Milano (IT); Bianco, Andrea, Costa Masnaga (LC) (IT); Cortecchia, Fausto, Faenza (RA) (IT); Diolaiti, Emiliano, Baricella (BO) (IT); Lessio, Lessio, Cittadella (PD) (IT); Lombini, Matteo, Bologna (IT); Macchi, Alberto, Milano (IT); Malaguti, Giuseppe, Poggio Renatico (FE) (IT); Millul, Rachele, Merate (LC) (IT); Pareschi, Giovanni, Milano (IT)
(74) Representative: Mati, Silvia

(57) **Abstract**

According to one aspect, the device for sanitizing air by ultraviolet radiation subject-matter of the present invention comprises a support base (10) and a support post (20) having an elongated shape along an own longitudinal axis (X), the support post (20) being associated with the support base (10) at its own lower end (21). The device (100) further comprises a sanitation module (300), associated with an upper end (22) of the post (20) and comprising a first body (30) comprising an upper portion (31) substantially shaped like a hollow spherical cap having a top portion (312) which abuts above the upper end (22) of the support post (20) and a substantially planar lower portion (32), said lower portion (32) being removably associated with the upper portion (31) at its base and defines, in conjunction with the upper portion (31), an internal volume in which there are arranged at least one ventilation device (33), at least one ultraviolet radiation generating device (34), at least one deflector element (35', 35", 35‴) and an emission duct (36); the sanitation module (300) further comprises a second body (40) placed below said first body (30) and comprising an upper surface (41) arranged facing the lower portion (32) of the first body (30) and spaced from it, the first body (30) and the second body (40) being made of opaque material with respect to ultraviolet radiation. Said at least one ventilation device (33) is arranged in association with the lower portion (32) of the first body (30) so as to have a suction mouth (331) facing the upper surface (41) of the second body (40) and the emission duct (36) is arranged at the top portion (312) of the upper portion (31) and has a substantially frustoconical shape which extends coaxially to the support post (20) and comprises a first section arranged inside the first body (30) and a second section (361), arranged outside the first body (30) and having a flared shape ending in the major base of the emission duct (36), said first section having a plurality of through openings (364).

## Description

### TECHNICAL FIELD

The present invention refers to the field of air sanitizing systems. Specifically, the invention refers to a device for sanitizing air by ultraviolet radiation.

### BACKGROUND

As is well known, in the field of the apparatuses for sanitizing fluids, particularly air, various technologies are used aimed at reducing the content of unwanted micro-organisms, such as viruses, bacteria, spores, moulds, or many others.

For example, devices are known that envisage the use of typically "gas-discharge" lamps that induce fluorescence in the mercury vapours, with consequent emission of electromagnetic radiation in the far ultraviolet (180-254 nm) as a result of which an ozone formation and cleavage mechanism through the oxygen contained in the fluid itself to be sanitized is created, increasing the disinfection efficiency through the chemical mechanism. Despite an effective sanitation against pathogenic micro-organisms present in the fluid subjected to such treatment is carried out, the release of ozone into the environment may nevertheless interact with organic compounds and materials present therein and cause a damage thereof. Furthermore, such devices are not suitable for use in environments where the presence of living beings (humans, animals or plants) is expected, unless the environment itself has been thoroughly ventilated.

Other known apparatuses subject the volume of air to be sanitized to ionizing radiations. Such systems must, however, include appropriate shielding systems that are not easy to implement, consequently entailing unwanted additional costs, in order to avoid a contamination of the surrounding environment by such radiations as they are potentially harmful to the health of subjects who may be exposed to them. This type of radiations can in fact lead to the development of skin tumours, or cataracts, as a consequence of the damage to the DNA molecules of the subjects exposed to them, due to the interaction with such radiation.

A known alternative type of devices suitable for carrying out a sanitation treatment of air and/or objects, that is generally more economically and effectively than the devices of the prior art described above, envisages the use of UV radiation sources, in particular of C-type, that is in the spectral range of wavelengths between 240 and 290 nm. These devices are generally used to carry out an efficient sanitation of the ambient air in different settings, such as hospitals, clinics, food and pharmaceutical industries, offices, shops, warehouses and others, or in all cases where it is necessary to provide that the inactivation of a certain percentage of pathogenic micro-organisms is constant over time at each instant.

In this context, devices are known that comprise a system for generating UV-C radiation (Mercury vapour fluorescent lamps or LEDs) arranged on a support structure, to be placed at an upper wall of the environment to be sanitized (for example a ceiling) so as to irradiate the upper layers of the air present in the environment itself. These systems make it possible to achieve a good compromise between the cost-effectiveness of the process carried out and the possibility of use in environments where living beings are intended to stay, if suitably provided with a UV-C shielding, but allow a little flexible use as they are constrained to a specific position within the environment in which they are located and therefore limited in the possible range of action.

In addition, environmental sanitation interventions are increasingly required in public spaces intended for the passage or for people to stay, such as shops, offices or school classrooms, entrance halls, stairs and lifts, and there is therefore a need to have a device for sanitizing air that is efficient and at the same time usable in the presence of living beings.

### OBJECTS AND SUMMARY OF THE INVENTION

An object of the present invention is to overcome the drawbacks of the prior art.

In particular, it is an object of the present invention to present a device for sanitizing air that is versatile, that is usable in various types of environments, in particular where the presence of living beings is expected.

Furthermore, the object of the present invention is to present a device for sanitizing air against pathogenic micro-organisms capable of carrying out an efficient sanitation of high volumes of air, while ensuring the safety of the users present in the vicinity of the device during its operation.

A further object of the present invention is to present a device for sanitizing air that adequately combines efficiency characteristics with aesthetic requirements.

It is also an object of the present invention to present a device that entails a lower environmental impact than the prior art systems, i.e. that envisages the reuse of elements or parts of structures that are disused or already destined for recycling or end-of-life.

Another object of the present invention is to present a device easily installable in the environment to be subjected to sanitation.

Finally, the object of the present invention is to present a device for sanitizing air having a contained encumbrance and in which the elements and the devices that compose it are easily accessible, so as to result in an easy assembly as well as cleaning and maintenance of the same.

These and other objects of the present invention are achieved by a device for sanitizing air by ultraviolet radiation incorporating the features of the appended claims, which form an integral part of this description.

According to one aspect, the device for sanitizing air by ultraviolet radiation which is the subject-matter of the present invention comprises a support base and a support post having an elongated shape along an own longitudinal axis, the support post being associated with the support base at its own lower end. The device further comprises a sanitation module, associated with an upper end of the post and comprising a first body comprising an upper portion substantially shaped like a hollow spherical cap having a top portion which abuts above the upper end of the support post and a substantially planar lower portion, said lower portion being removably associated with the upper portion at its base and defines, in conjunction with the upper portion, an internal volume in which there are arranged at least one ventilation device, at least one ultraviolet radiation generating device, at least one deflector element and an emission duct; the sanitation module further comprises a second body placed below said first body and comprising an upper surface arranged facing the lower portion of the first body and spaced from it, the first body and the second body being made of opaque material with respect to ultraviolet radiation. Said at least one ventilation device is arranged in association with the lower portion of the first body so as to have a suction mouth facing the upper surface of the second body and the emission duct is arranged at the top portion of the upper portion and has a substantially frustoconical shape which extends coaxially to the support post and comprises a first section arranged inside the first body and a second section, arranged outside the first body, and having a flared shape ending in the major base of the emission duct, said first section having a plurality of through openings.

According to this solution, a versatile device that allows carrying out an efficient sanitation of the air injected into it in a short time and in a cost-effective manner and which at the same time guarantees safety of use in the presence of living beings is realized.

In one embodiment, an internal surface portion of the upper portion and at least an upper surface portion of the lower portion of said first body, is of reflective type.

In this way, a cavity is realized within which the reflection of the UV-C rays is not attenuated and the UV-C rays are only weakly absorbed; there is therefore an effect of amplification of the luminous flow to which the volume of air passing inside the cavity is subject at a given instant, with the effect of enhancing the sanitizing action carried out on the flow of air crossing this cavity.

Preferably the value of the main dimension of the upper surface of the second body is greater than the value of the main dimension of a lower surface of the lower portion of the first body.

According to this configuration, the leakage of any ultraviolet radiation C towards the zones that are located below the second body is advantageously prevented, and therefore the safety of use of the device is guaranteed, even in the event of a partial diffusion of radiation through the ventilation devices towards the external environment.

Preferably, the lower portion of the first body has one or more housing seats comprising shaped openings made in the body of the lower portion, each housing seat being configured to accommodate a respective ventilation device arranged so as to have a suction mouth facing the upper surface of the second body, so as to arrange the internal volume of the first body in fluid connection with the external environment.

Preferably, the lower portion of the first body is shaped according to a discoidal plate, preferably circular, having an external diameter whose value is between about 40 cm and about 140 cm and arranged according to a plane substantially perpendicular to the axis of symmetry of the support post.

Preferably, the second body comprises a plate-like discoidal element, preferably circular, having an upper surface facing the lower surface of the lower portion of the first body, and which extends on a plane substantially perpendicular to the longitudinal axis of the support post, said plate-like discoidal element having an external diameter whose value is between about 60 cm and about 160 cm.

Preferably, the length of the second section of the emission duct has a value greater than or equal to about 5 cm and less than or equal to about 30 cm.

This ensures a correct and efficient diffusion of the flow of sanitized air in the environment in which the sanitizing device is operating.

In one embodiment, the first body comprises a first cylindrical sleeve extending vertically from the lower portion, and which is configured so as to have an internal diameter such as to engage on the body of the support post to which it is connected by means of fixing means.

This solution advantageously allows both assembly and maintenance operations to be carried out easily and therefore efficiently and economically.

Preferably, the sanitation module comprises two ultraviolet radiation generating devices, associated with the support post or with the first cylindrical sleeve, the one in a diametrically opposite position with respect to the other, preferably oriented so as to emit the radiation according to a direction perpendicular to the longitudinal axis of the support post, towards the internal volume of the first body.

According to this configuration it is possible to achieve a homogeneous diffusion of the radiation emitted in the internal volume of the first body and therefore an efficient sanitation of the air contained therein.

Preferably, the ultraviolet radiation generating device is configured to emit C-type ultraviolet radiation having a wavelength λ between 240 and 290 nm.

In this way, the radiation emitted by the light device is not only non-ionizing, but also prevents the production of ozone (which could only occur at shorter wavelengths). Since the UV-C light remains mainly inside the cavity where the light source and the ventilation system are contained, with very low dispersion towards the outside, the device is advantageously usable in environments where the presence of living beings is expected.

In one embodiment, the sanitation module comprises at least one filtration device, located at least at the suction mouth of each ventilation device.

Thanks to this solution, the efficiency of the sanitation process is increased as the air coming from the environment is previously filtered and purified before being treated by the ultraviolet radiation generating device.

According to an embodiment, the plate-like discoidal element is removably connected to a box-shaped element arranged below it, which is connected to a second cylindrical sleeve that allows the association thereof to the support post.

Preferably, the box-shaped element of the second body is substantially shaped like a hollow spherical cap, having its base in mutual contact with the plate-like discoidal element.

In one embodiment, the sanitizing device is provided with a timing device, configured to adjust the operating time of the ventilation devices and/or of the ultraviolet light generating device.

This solution increases the usability of the sanitizing device by users insofar as it makes possible a versatile programming of the sanitizing cycles, i.e. of the frequency and duration of the sanitizing operations carried out, optimizing their performance based on the specific needs of the user.

Further features and advantages of the present invention will be more evident from the description of the accompanying drawings.

### BRIEF DESCRIPTION OF THE DRAWINGS

The invention is described hereinbelow with reference to certain examples provided by way of non-limiting example and illustrated in the accompanying drawings. These drawings illustrate different aspects and embodiments of the present invention and reference numerals illustrating structures, components, materials and/or similar elements in different drawings are indicated by similar reference numerals, where appropriate. Moreover, for clarity of illustration, certain references may not be repeated in all drawings.
Figure 1 is a side schematic view of a device for sanitizing air according to an embodiment of the present invention;
Figure 2 is a side schematic view of the device of Figure 1, with some parts omitted to better highlight others;
Figure 3 is a side schematic view of a detail of the device of Figure 1;
Figure 4 is a perspective schematic view of the element of Figure 3;
Figure 5 is a perspective schematic view from above of the device in Figure 1;
Figure 6 is a schematic view from above of the device of Figure 1; and
Figure 7 is a perspective schematic view from below of the device of Figure 1.

### DETAILED DESCRIPTION OF THE INVENTION

While the invention is susceptible to various modifications and alternative constructions, certain preferred embodiments are shown in the drawings and are described hereinbelow in detail. It must in any case be understood that there is no intention to limit the invention to the specific embodiment illustrated, but, on the contrary, the invention intends covering all the modifications, alternative and equivalent constructions that fall within the scope of the invention as defined in the claims.

The use of "for example", "etc.", "or" indicates non-exclusive alternatives without limitation, unless otherwise indicated. The use of "includes" means "includes, but not limited to" unless otherwise indicated.

In the present description and in the subsequent claims, the term "main dimension of a surface" is intended to indicate the greatest distance among the distances that separate any pair of points belonging to the surface itself.

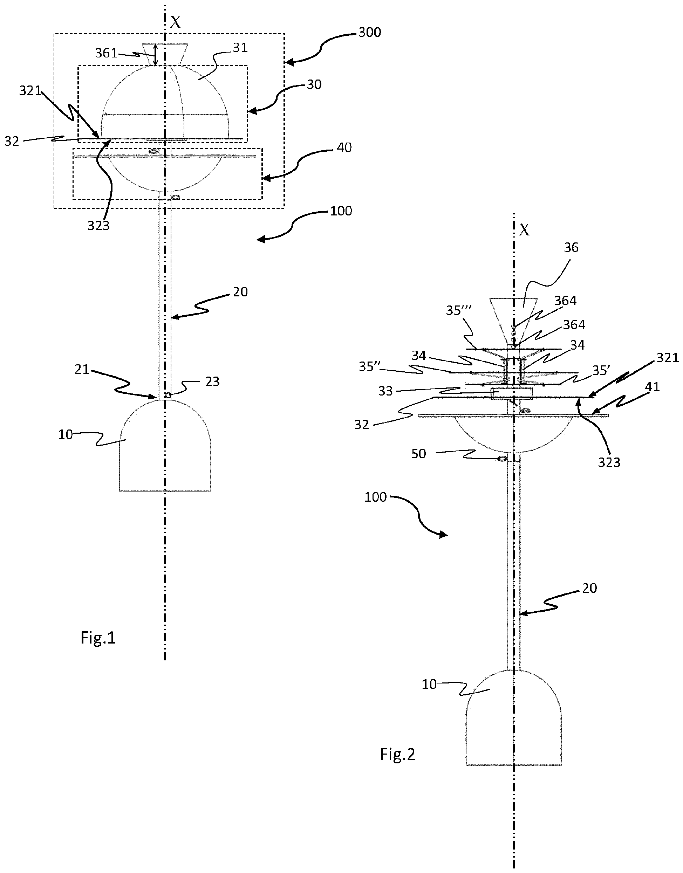

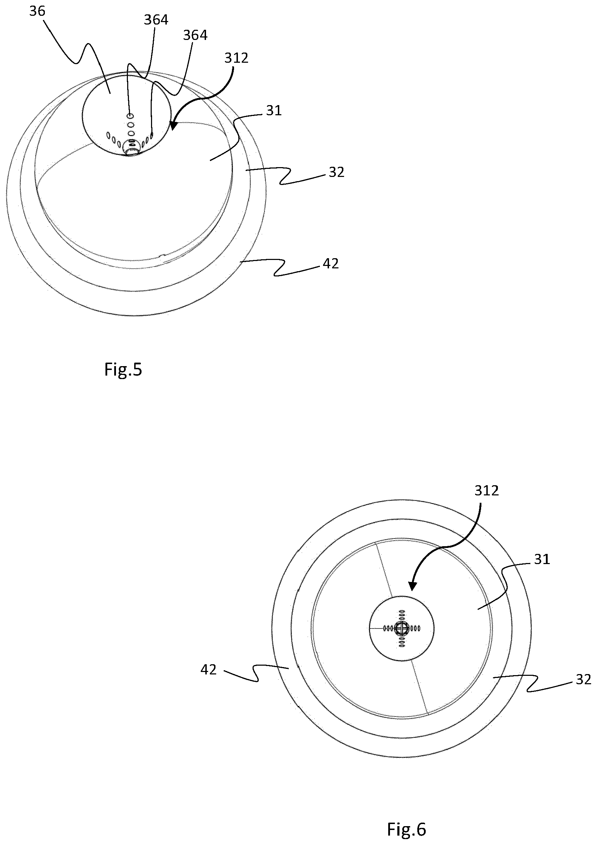

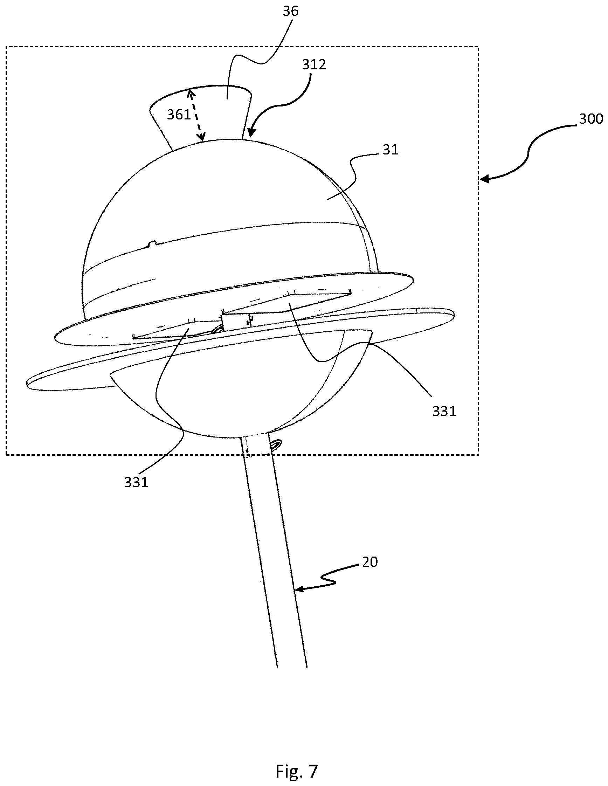

With reference to Figures 1 - 7, a device 100 for sanitizing air by ultraviolet radiation is described, according to a preferred embodiment of the invention.

The device 100 for sanitizing air comprises a support base 10, a post 20 and a sanitation module 300.

The post 20 has a preferably tubular shape, elongated along an own longitudinal axis X, and is associated with the support base 10 at its own lower end 21.

Preferably, the support post 20 is made of a robust material configured to withstand stresses, even of considerable intensity, without bending or breaking. Likewise, the support base is made of robust material, such as stone, metal, cement material or rigid plastic possibly also in the form of a container adapted to accommodate a liquid inside it, and is shaped in such a way as to guarantee the stability of the device 100.

The sanitation module 300 is adapted to carry out a sanitation treatment of an air flow that is made to flow through it and comprises a first body 30 and a second body 40 both connected to the support post 20 and arranged along it, so that the second body 40 is positioned below the first body 30.

Preferably, the first body 30 is entirely made of material opaque to ultraviolet light and comprises an upper portion 31 which is substantially shaped like a hollow spherical cap, having a top portion 312 which abuts above the upper end 22 of the support post 20. The first body 30 further comprises a substantially planar lower portion 32, which is removably associated with the upper portion 31 at its base; the lower portion 32 in conjunction with the upper portion 31 define an internal volume in which one or more ultraviolet radiation generating devices 34 and at least one ventilation device 33 are housed.

Preferably, the lower portion 32 of the first body 30 is shaped according to a discoidal plate, preferably circular, for example according to a flat circular crown having an external diameter between about 40 cm and about 140 cm and arranged according to a plane substantially perpendicular to the longitudinal axis X of the support post 20.

According to a preferred embodiment, the lower portion 32 has a through hole (not shown in the figures) obtained at its centre of symmetry, and the first body 30 comprises a first cylindrical sleeve 37 connected to the lower portion 32 at said through hole. Preferably said first cylindrical sleeve 37 extends from the lower portion 32 vertically and is configured so as to have an internal diameter substantially corresponding to the diameter of said through hole and such as to engage on the body of the support post 20, and is configured so as to be fixed to it by means of fixing means 50.

In accordance with the invention, the second body 40 may be variously shaped, being in any case made of opaque material with respect to ultraviolet radiation and comprising an upper surface 41 arranged facing the lower portion 32 of the first body 30 and spaced from it. Preferably, the second body 40 comprises a plate-like discoidal element 42, preferably circular, having an external diameter whose value is between about 60 cm and about 160 cm, which extends on a plane substantially perpendicular to the longitudinal axis X of the support post 20, therefore parallel to the lower portion 32 of the first body 30, and whose upper surface coincides with the upper surface 41 of the second body 40.

Preferably, the plate-like discoidal element 42 is arranged at a substantially constant distance from the lower portion 32 of the first body 30, with which together it defines a zone within which ambient air can circulate freely.

According to a preferred configuration, the plate-like discoidal element 42 has a through hole (not shown in the figures) obtained at its centre of symmetry, and the second body 40 comprises a second cylindrical sleeve 44 arranged at said through hole. Preferably said second cylindrical sleeve 44 has an internal diameter substantially equivalent to the diameter of said through hole and such as to engage on the body of the support post 20, and is configured to be fixed to it by means of fixing means 50.

According to a preferred embodiment, the plate-like discoidal element 42 is removably connected to a box-shaped element 43 arranged below it, which is connected to the second cylindrical sleeve 44 that allows the association thereof to the support post 20.

According to a preferred configuration, the lower portion 32 of the first body 30 has one or more housing seats 322, each of which is configured to accommodate a respective ventilation device 33 which is configured to draw air from the environment and to convey it into the internal volume of the first body 30. Preferably, the housing seats 322 comprise suitable shaped openings, made in the body of the lower portion 32 in such a way as to arrange the internal volume of the first body 30 in fluid connection with the external environment, through the ventilation device 33 which is respectively arranged at each of these seats 322. More particularly, each ventilation device 33 is arranged in association with the lower portion 32, through the mutual housing seat 322, so as to have a suction mouth 331 facing the upper surface 41 of the second body 40.

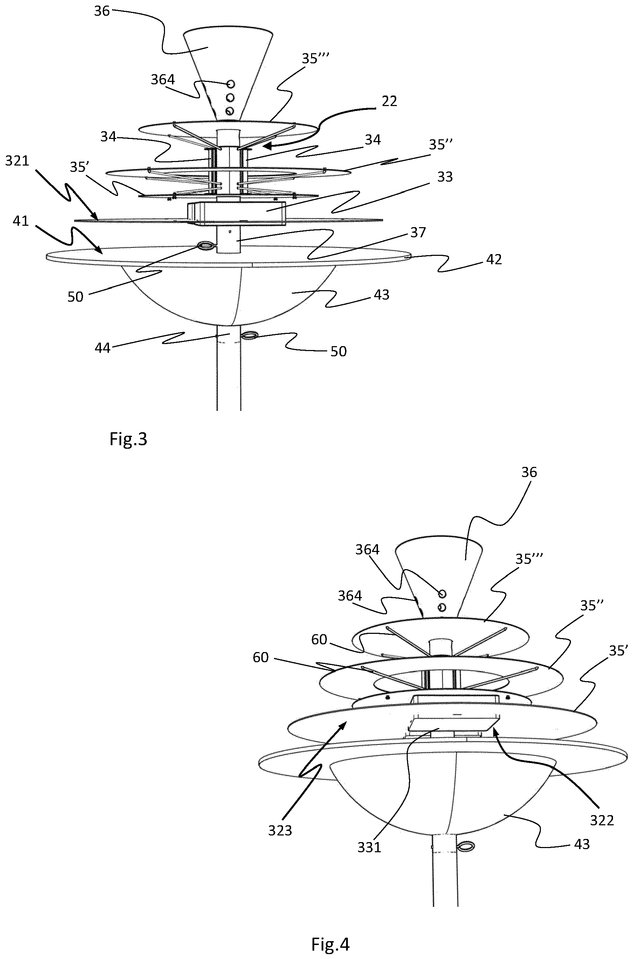

According to a preferred embodiment, such as for example shown in Figures 2 and 3, the sanitation module 300 comprises two ventilation devices 33; still more preferably the two ventilation devices 33 are arranged in a position symmetrically opposite the longitudinal axis X of the support post 20.

As mentioned, the sanitation module 300 further comprises at least one ultraviolet radiation generating device 34, preferably removably connected to the body of the support post 20 or, in a preferred configuration, to the first cylindrical sleeve 37. Preferably, the ultraviolet radiation generating device 34 is connected to the first cylindrical sleeve 37 by means of fixing means (not shown in the figures) that fit into suitable seats obtained in the body of the first sleeve 37 itself, such as for example eyelet holes.

According to a preferred embodiment, such as for example shown in Figures 2 and 3, the sanitation module 300 comprises two ultraviolet radiation generating devices 34, associated with the first cylindrical sleeve 37 the one in a diametrically opposite position with respect to the other, preferably oriented so as to emit radiation according to a direction perpendicular to the longitudinal axis X of the post 20, towards the internal volume of the first body 30.

According to the invention, the ultraviolet radiation generating device 34 emits C-type ultraviolet radiation and preferably consists of a mercury fluorescent lamp, which is adapted to emit a radiation having a wavelength λ whose value is about 253 nm. This wavelength value advantageously allows operating on the nucleic acids and the proteins of the bacteria and/or viruses present in the air to be sanitized, acting on the nucleic acid bonds.

According to an alternative embodiment, the ultraviolet radiation generating devices 34 consist of LEDs (Light Emitting Diode), and adapted to emit a radiation having a wavelength λ whose value is between about 240 nm and about 290 nm, therefore advantageously of a non-ionizing type.

The sanitation module further comprises an emission duct 36 arranged at the top portion 312 of the upper portion 31, and connected to the first cylindrical sleeve 37 at the upper end 21 of the support post 20; said emission duct 36 is substantially shaped according to a truncated cone, preferably coaxial to the support post 20 as well as to the first sleeve 37. The emission duct 36 extends along the longitudinal axis X of the post 20 in such a way as to have its major base above its minor base (not indicated in the figures), that is to say having an upwardly flared profile ending in its major base, which advantageously allows to guarantee an effective emission of the sanitized air flow into the environment in which the device 100 is operating.

More particularly, the emission duct 36 comprises a first section (not identified in the figures) arranged inside the first body 30 and a second section 361, arranged outside the first body 30, and ending in the major base. According to a preferred embodiment, the first section has a plurality of through openings 364 adapted to arrange the internal volume of the first body 30 in fluid communication with the environment external to it. Preferably, the length of the second section 361 of the emission duct 36 has a value greater than or equal to about 5 cm and less than or equal to about 30 cm. This ensures a correct and efficient diffusion of the sanitized air flow in the environment.

According to a preferred configuration, the sanitation module 300 comprises at least one deflector element 35', 35", 35'" arranged inside the first body 30 and configured to optimize the path of the air flow in the internal volume thereof; the geometric shape of the deflector elements employed, as well as their quantity, are suitably configured based on the type of the ventilation device 33 used in the specific embodiment (for example of radial, tangential, centrifugal, or axial type).

According to the preferred embodiment of the invention shown in Figures 1 to 7, the sanitation module comprises two ventilation devices 33 and three deflector elements 35', 35", 35‴ having a substantially plate-like shape. Preferably, the ventilation devices 33 consist of fans of radial type; in this way, the flow of air sucked in from the external environment through the suction vents 331 is efficiently conveyed inside the first body 30 and uniformly irradiated by the ultraviolet radiation emitted by the generating devices 34.

More in detail, a first deflector element 35' is shaped like a discoidal plate, preferably circular, and is associated with the first sleeve 37, so as to be arranged substantially perpendicular to the longitudinal axis X of the post itself. According to a preferred configuration, said first deflector element 35' has a through hole at its centre of symmetry, in which the first sleeve 37 is engaged and is connected to the latter by means of rod-like connection means 60 so as to achieve, in a simple and at the same time stable way, a connection between these elements while minimizing the encumbrance of the elements suitable for achieving it.

The first deflector element 35' is preferably made of material opaque to ultraviolet light and is advantageously arranged, along the vertical development of the support post 20, above the ventilation devices 33.

According to a preferred configuration, the first deflector element has discoidal geometry. Preferably, the first deflector element 35' is configured so that the value of its external diameter is at least equal to 90% of the maximum dimension of the area identified by the encumbrance of the ventilation devices 33 arranged on the lower portion 32 of the first body 30; even more preferably the value of the external diameter of the first deflector element 35' is such that the latter overlaps entirely with the free passage gap (not shown in the figures) of each ventilation device 33, which puts the internal volume of the body 30 in flow communication with the external environment. In this way, the air flow injected by the ventilation devices 33 inside the body 30 is correctly diffused into the internal volume thereof and at the same time unwanted dispersions of ultraviolet radiation towards the external environment are advantageously avoided.

The device 100 further comprises a second deflector element 35" arranged along the vertical development of the support post 20, above the first deflector element 35'. Preferably, the second deflector element 35" has an annular plate-like shape, preferably of circular shape, and is associated with the support post 20 so as to be arranged substantially perpendicular to the longitudinal axis X of the post itself.

According to a preferred configuration, said second deflector element 35" is connected to the first sleeve 37 similarly to what is provided for the first deflector element 35', i.e. by means of rod-like connection means 60, and is arranged so that its centre of symmetry is approximately on the longitudinal axis X of the support post 20.

Preferably, the second deflector element 35" is arranged substantially parallel to the first deflector element 35', at a substantially constant distance from the latter.

The device 100 further comprises a third deflector element 35'" arranged along the vertical development of the support post 20, above the second deflector element 35". This element has a shape similar to that of the first deflector element 35', i.e. shaped like a discoidal plate, preferably of circular shape, and is associated with the first sleeve 37 so as to be arranged substantially perpendicular to the longitudinal axis X of the post itself and parallel to the first 35' and the second 35" deflector element. The third deflector element 35‴ preferably consists of material opaque to ultraviolet light so as to prevent the dispersion of ultraviolet radiation towards the external environment, for example through the through openings 364 of the emission duct 36. Preferably, the third deflector element 35'" has a through hole at its centre of symmetry, in which the first sleeve 37 engages and is connected to the latter by means of rod-like connection means 60.

The connection means between deflector elements 35', 35", 35‴ and first sleeve 37 may possibly be shaped differently from what is provided, with the precaution of allowing an efficient circulation of the air flow inside the internal volume of the first body 30 and at the same time achieving a stable connection with the first sleeve 37 itself.

According to a preferred embodiment, at least an internal surface portion (not shown in the figures) of the upper portion 31, i.e. the surface facing the internal volume of the first body 30, is of reflective type. This internal surface can, for example, be partially or fully coated with a reflective material, e.g. by realizing a coating with fluoropolymers, or with Barium sulphate, or in another material having high reflective properties with respect to the UV-C band.

According to a preferred embodiment, at least an upper surface portion 321 of the lower portion 32 of said first body 30, is of reflective type.

Preferably, the value of the main dimension of the upper surface 41 of the second body 40 is greater than the value of the main dimension of a lower surface 323 of the lower portion 32 of the first body 30.

The sanitation module 300 may further advantageously comprise at least one filtration device (not shown in the figures), placed at least at the suction mouth 331 of each ventilation device 33. The filtration device can be variously configured, in relation to the specific needs of the user, for example by providing for a filtration against dust, and/or a filtration against viruses and bacteria.

The sanitizing device 100 is further provided with a power supply cable (not shown in the figures), preferably arranged inside the body of the support post 20 and emerging therefrom through a through hole 23 obtained on it, and with a switch (not shown in the figures) adapted to operate the ultraviolet light generating device 34 and/or the ventilation device 33.

The sanitizing device 100 may further be provided with a timing device (not shown in the figures), configured to adjust the operating time of the ventilation devices 33 and/or of the ultraviolet light generating device 34. Additionally, a potentiometer (not shown in the figures) adapted to adjust the speed of the ventilation devices 33 can be used.

With particular regard to the operation of the device 100, after switching on by means of a switch, a flow of air is drawn from the environment through at least one ventilation device 33, in particular at the interface zone between the lower portion 32 of the first body 30 and the upper surface 41 of the second body 40. Again thanks to the action of at least one ventilation device 33, the air flow is then conveyed towards the internal volume of the first body 30 and irradiated by means of an ultraviolet radiation, preferably of C-type, emitted by one or more ultraviolet radiation generating devices 34; this radiation is preferably characterized by a wavelength *λ*, whose value is between about 240 nm and about 290 nm.

The particular arrangement of the ventilation devices 33, together with the presence of at least one deflector element 35, makes it possible to optimize the path of the air flow inside the first body 30, so as to ensure an adequate time of exposure of the flow to the radiation emitted by one or more generating devices 34 and ensure adequate sanitation.

The action of the ventilation devices 33 also makes it possible to push the sanitized air into the emission duct 36, through the through openings 364 present on the body of the same and therefore to be released into the environment.

The invention thus conceived is susceptible to several modifications and variations, all falling within the scope of the inventive concept.

For example, the box-shaped element 43 of the second body 40 is substantially shaped like a hollow spherical cap, possibly provided with a plurality of through holes adapted to aerate it, having its own base in mutual contact with the plate-like discoidal element 42. According to this configuration, a shape harmony with the first body 30 is achieved, so that the sanitizing device 100 as a whole is made aesthetically pleasing and therefore conveniently usable in every type of environment, especially where users are expected to stay.

Moreover, the box-shaped element 43, thanks to its hollow shape and to the fact that it is removably connected to the plate-like discoidal element 42, can be easily used to house any circuitry, such as, for example, the elements adapted to power the LEDs (if their use is envisaged as generating devices 34) or a possible programmable control unit adapted to control the sanitizing device 100.

Moreover, all the details can be replaced by other technically equivalent elements.

In practice, the materials used, as well as the contingent shapes and sizes, can be whatever according to the requirements without for this reason departing from the scope of protection of the following claims.

## Claims

1. Device (100) for sanitizing air by ultraviolet radiation comprising:
- a support base (10);
- a support post (20) having an elongated shape along an own longitudinal axis (X), the support post (20) being associated with the support base (10) at its own lower end (21);
- a sanitation module (300), associated with an upper end (22) of the post (20) and comprising:
- a first body (30) comprising an upper portion (31) substantially shaped like a hollow spherical cap having a top portion (312) which abuts above the upper end (22) of the support post (20) and a substantially planar lower portion (32), said lower portion (32) being removably associated with the upper portion (31) at its base and defines, in conjunction with the upper portion (31), an internal volume in which there are arranged at least one ventilation device (33), at least one ultraviolet radiation generating device (34), at least one deflector element (35', 35", 35‴) and an emission duct (36);
- a second body (40) placed below said first body (30) and comprising an upper surface (41) arranged facing the lower portion (32) of the first body (30) and spaced from it, the first body (30) and the second body (40) being made of opaque material with respect to ultraviolet radiation; wherein
- said at least one ventilation device (33) is arranged in association with the lower portion (32) of the first body (30) so as to have a suction mouth (331) facing the upper surface (41) of the second body (40); and wherein
- the emission duct (36) is arranged at the top portion (312) of the upper portion (31) and has a substantially frustoconical shape which extends coaxially to the support post (20) and comprises a first section arranged inside the first body (30) and a second section (361), arranged outside the first body (30) and having a flared shape ending in the major base of the emission duct (36), said first section having a plurality of through openings (364).

2. Device (100) according to claim 1, wherein at least an internal surface portion of the upper portion (31) and at least an upper surface portion (321) of the lower portion (32) of said first body (30), is of reflective type.

3. Device (100) according to claim 1 or 2, wherein the value of the main dimension of the upper surface (41) of the second body (40) is greater than the value of the main dimension of a lower surface (323) of the lower portion (32) of the first body (30).

4. Device (100) according to any one of claims 1 to 3, wherein the lower portion (32) of the first body (30) has one or more housing seats (322) comprising shaped openings made in the body of the lower portion (32), each housing seat (322) being configured to accommodate a respective ventilation device (33) arranged so as to have a suction mouth (331) facing the upper surface (41) of the second body (40), so as to arrange the internal volume of the first body (30) in fluid connection with the external environment.

5. Device (100) according to any one of claims 1 to 4, wherein the lower portion (32) of the first body (30) is shaped according to a discoidal plate, preferably circular, having an external diameter whose value is between about 40 cm and about 140 cm, and arranged according to a plane substantially perpendicular to the axis of symmetry (X) of the support post (20).

6. Device (100) according to any one of claims 3 to 5, wherein the second body (40) comprises a plate-like discoidal element (42), preferably circular, having an upper surface (41) facing the lower surface (323) of the lower portion (32) of the first body (30), and which extends on a plane substantially perpendicular to the longitudinal axis (X) of the support post (20), said plate-like discoidal element (42) having an external diameter whose value is between about 60 cm and about 160 cm.

7. Device (100) according to any one of claims 1 to 6, wherein the length of the second section (361) of the emission duct (36) has a value greater than or equal to about 5 cm and less than or equal to about 30 cm.

8. Device (100) according to any one of claims 1 to 7, wherein the first body (30) comprises a first cylindrical sleeve (37) extending vertically from the lower portion (32), and which is configured so as to have an internal diameter such as to engage on the body of the support post (20) to which it is connected by means of fixing means (50).

9. Device (100) according to claim 1 or 8, wherein the sanitation module (300) comprises two ultraviolet radiation generating devices (34), associated with the support post (20) or with the first cylindrical sleeve (37), the one in a diametrically opposite position with respect to the other, preferably oriented so as to emit radiation according to a direction perpendicular to the longitudinal axis (X) of the support post (20), towards the internal volume of the first body (30).

10. Device (100) according to any one of the preceding claims, wherein the ultraviolet radiation generating device (34) is configured to emit C-type ultraviolet radiation having a wavelength (*λ*) whose value is between about 240 nm and about 290 nm.
